# EUROPEAN PATENT SPECIFICATION

(11) **EP 0 740 504 B1**
(45) Date of publication and mention of the grant of the patent: **02.01.2003**
(21) Application number: 95908573.9
(22) Date of filing: 18.01.1995
(51) Int. Cl.: A01H 5/02, A01H 1/02, C12N 15/29, C12N 15/82

(54) **PHYTOPHTHORA RESISTANCE GENE OF CATHARANTHUS AND ITS USE**
PHYTOPHTHORA RESISTENZGEN VON CATHARANTHUS UND DESSEN VERWENDUNG
GENE DE CATHARANTUS RESISTANT AU PHYTOPHTHORA ET SON UTILISATION

(30) Priority: 21.01.1994 US 184319
(43) Date of publication of application: 06.11.1996
(73) Proprietor: Goldsmith Seeds Inc., Gilroy, CA 95020 (US)
(72) Inventor: BOWMAN, Robert M., Gilroy, CA 95020 (US)
(74) Representative: Goldin, Douglas Michael
(86) International application number: PCT/US95/00730
(87) International publication number: WO 95/019698

(56) References cited:
- EP-A- 0 200 225
- WO-A-93/19188
- US-A- 4 594 810
- PATENT ABSTRACTS OF JAPAN vol. 017 no. 678 (C-1141) ,13 December 1993 & JP,A,05 227858 (DAICEL CHEM IND LTD) 7 September 1993,

## Description

### Technical Field

The present invention relates to a *Catharanthus* resistance gene, *Catharanthus* seed, a *Catharanthus* plant, a *Catharanthus* variety and a *Catharanthus* hybrid which contain a level of resistance to the disease *Phytophthora parasitica.* The Phytophthora resistance gene of the present invention can be incorporated into various *Catharanthus* genetic backgrounds and also into other genera. The present invention also relates to a *Catharanthus* plant product, an increased level of alkaloid content, and an increased resistance to certain insects.

### Background of the Invention

*Catharanthus roseus* (L.) G. Don, also called periwinkle, or vinca, originates from Madagascar and belongs to the family of the Apocynaceae. This species is frequently grown annually from seed or cuttings in temperate climates for use in summer bedding or as a pot plant for the conservatory or the windowsill. *C*. *roseus* has long been grown as an ornamental in tropical regions of the world. As a consequence of its self-seediness (ability to pollinate itself and readily form mature selfed seed) it is now widely naturalized in many tropical regions. Commonly known as Madagascar periwinkle or vinca (not to be confused with the separate genus *Vinca)* this ornamental is commonly propagated from seed and less frequently from stem cuttings.

Periwinkle is valued for its bushy habit, large desirable flowers, tolerance to heat, drought and direct sunlight. In addition to its horticultural merits, *C*. *roseus* contains alkaloids used to retard certain diseases, such as leukemia.

*Catharanthus* species are well known for their production of indole alkaloids as described in Farnsworth, Lloydia 24:105-139 (1961); Sevestre-Rigouzzo, et al., Euphytica 66:151-1569 (1993). *Catharanthus roseus* is one of the most thoroughly investigated of plants with regard to its constituent indole alkaloids, of which more than 70 have been isolated from the whole plant as described in Balsevich and Hogge, J. Nat. Prod., 51:1173-1177 (1988). Alkaloids are generally known as compounds biologically active against pathogens and herbivores as discussed in vanDam, et al., Oecologia 95:425-430 (1993). Indole alkaloids are potent antifeedants. Tested in a bio-assay using a 0.04% solution, vinblastine and catharanthine appeared to be the most deterrent alkaloids against the polyphagous *Spodoptera* larvae as described in Meisner, et al., J. Econ. Entomol. 74:131-135 (1981); Chockalingam, et al., J. Environ. Biol. 10:303-307 (1989). Periwinkle extracts have also been shown to have strong inhibitory activity against several bacterial genera. Famsworth Lloydia 24:105-139 (1961) also described the anthelmintic activity of alkaloid fractions of *C. roseus.* Alkaloids obtained from *C. roseus* provide the basis of a 22-year old industry yielding well over a hundred million dollars annually as described in Raven, Diversity 9:49-51 (1993). The anticancer activity of vinblastine and vincristine, both isolated from *C. roseus*, is well documented in the pharmaceutical industry.

To date there has been no known resistance to *Phytophthora* in *Catharanthus* species. The principle problem with growing periwinkle is its sensitivity and susceptibility to attack by *Phytophthora parasitica.* Stem and crown rot caused *by Phytophthora parasitica* is a common problem on *Catharanthus.* Symptoms typically are associated with the final stages of disease development, but infected plants can support a population of the pathogen without showing visual symptoms.

Keen and Yoshikawa (in Erwin et al.) 279-284 (1983) describe natural mechanisms of resistance to *Phytophthora* spp. in other crops. General resistance mechanisms against *Phytophthora* spp. include structural features of the host, preformed chemical inhibitors, induced structural barriers, hypersensitive reactions and phytoalexins. Keen, Adv. Plant Pathol. 65:35-82 (1982) also suggests that specific resistance to *Phytophthora* spp. is usually controlled by single host resistance genes. Monogenetically inherited resistance to different species of *Phytophthora* has been reported in several crops other than *Catharanthus.* Resistance has most often been found to be attributable to single, dominant alleles (Umaerus et al., in Erwin et al., 315-326 (1983). Colon et al., Euphytica 55-64 (1983) described resistance to *Phytophthora infestans in Solanum* spp.

The chemical metalaxyl (which is the active ingredient of the eumycete-specific fungicides Subdue and Ridomil) has been the primary fungicide used by nursery personnel to control stem and crown rots caused by *Phytophthora spp.* as discussed in Ferrin, et al. Plant Disease, Vol. 76, p. 60-63, p. 82-84 (1992). While some control of the diseases is effected by the chemical metalaxyl, Ferrin, supra has found one isolate of *P*. *parasifica* from a southern California nursery which was insensitive to metalaxyl. Tolerance to metalaxyl was expressed in vivo by the isolate chosen by Ferrin, et al. supra. Furthermore, this metalaxyl-tolerant isolate appears to be as virulent as sensitive wild-type isolates. For nurseries where plants are grown in containers, widespread failures in disease control would not necessarily be expected immediately after the appearance of metalaxyl tolerance because of the low frequency with which such tolerance appears and the time needed for the pathogen population to increase and be dispersed. Widespread disease control failures occur only after a large part of the pathogen population has become tolerant to the pesticide. The rate at which this metalaxyl-tolerant population becomes established depends largely on the stability of the pesticide tolerance, the selection pressure exerted on the pathogen population, and the ability of the pathogen to disperse. Because species of *Phytophthora* and *Pythium* have been detected in recycled irrigation water in nurseries in California, the requirement that commercial nurseries in certain areas of California trap and recycle all runoff water greatly increases the risk of recycling fungicide-tolerant populations of these pathogens in those nurseries. Thus, the appearance of metalaxyl tolerance could eventually result in control failures if the treatment of recirculated water is not sufficient to eliminate propagules of *Phytophthora spp.* and the selection pressure due to the continued use of the fungicide metalaxial is maintained.

The continuous use of metalaxyl as the primary means of controlling diseases caused by *Phytophthora* increases the likelihood for the development of insensitivity to metalaxyl. (Ferrin et al., supra) History has shown new isolates of *P*. *parasitica* insensitive to new fungicides eventually will evolve and since the use of chemicals can have side effects on people using the chemicals and the environment, it is highly desirable to use a *Catharanthus roseus* having genetic resistance to *Phytophthora spp.*

A genetic resistance to *Phytophthora* in periwinkle, if available, could be used to reduce or eliminate the use of the chemical metalaxyl and result in increased cost efficiencies and environmental safety.

### Summary of the Invention

The present invention relates to a *Catharanthus* seed, a *Catharanthus* plant, a *Catharanthus* variety, a *Catharanthus* hybrid and a method for producing a *Catharanthus* plant.

More specifically, the invention relates to a *Catharanthus* plant having resistance to the fungal disease *Phytophthora parasitica.* The present invention also relates to a *Catharanthus* plant having a moderate to high level of resistance to aphids *(Myzus persicae)* and mites *(Tetranychus urticae)*, and a *Catharanthus* plant having resistance to *Phytophthora parasitica* and an increase in total alkaloid content.

In one such embodiment, the present invention is directed to a *Catharanthus* plant or seed with a total alkaloid content between about 2.0% and about 9.5%.

The present invention further relates to a method of producing the disclosed *Catharanthus* plants and seeds by crossing a *Phytophthora* resistant plant of the instant invention with another *Catharanthus* plant. The invention also relates to the transfer of the genetic *Phytophthora* resistance into genera other than *Catharanthus,* including but not limited to the following genera: *Solanum, Capsicum, Eucalyptus, Carica, Ananas, Fragaria, Camellia, Castanea, Persea,* and *Citrus.*

The present invention further relates to a periwinkle plant having a level of resistance to certain insects, including but not limited to aphids *(Myzus persicae)* and mites *(Tetranychus urticae)*.

The present invention further relates to a periwinkle plant having increased resistance to *Phytophthora parasitica* which has a total alkaloid content of at least 2.0 percent or greater.

### Brief Description of the Drawings

In the accompanying drawings,
Figure 1 is a schematic perspective view of a periwinkle plant along wherein the numbers 1-10 indicate ratings for various levels of Phytophthora resistance or sensitivity.
Figure 2A, 2B, and 2C show schematically stages of the method of inoculation of the Catharanthus plant with the Phytophthora inoculum.

### Detailed Description of the Invention

In order to provide an understanding of several of the terms used in the specification and claims, the following definitions are provided:

Total alkaloid content - The term 'total alkaloid content' is intended to refer to the total alkaloid content expressed as a percent of total dry weight in the plant tissue.

Homogeneous Assemblage - The term 'homogeneous assemblage' is intended to refer to a group of seeds or plants which are homogeneous for a given periwinkle characteristic. This term is intended to include any seeds or plants which are homogeneous for either of the traits of *Phytophthora* resistance or total alkaloid content.

Plant Product - The term 'plant product' is intended to refer to plant extracts and plant derived chemical compounds or structures including alkaloids.

*Catharanthus roseus* (L) G. Don, also called periwinkle and vinca is primarily a self-pollinating species. The *Catharanthus roseus* of the present invention reproducibly expresses resistance to *Phytophthora parasitica*. We have isolated from our breeding populations a transferrable gene which conveys resistance to *Phytophthora*. This disease resistance trait has been expressed in many different genetic backgrounds of periwinkle.

To date, there is no known resistance to *Phytophthora* in any commercial cultivar of periwinkle except for the present invention. Additionally, there are no known reports of resistance to *Phytophthora* in any *Catharanthus* species, cultivar, in the wild or commercially available. All available cultivars and naturalized collections from the U.S., Mexico, Central America, Africa and Madagascar, have been tested and no indication of *Phytophthora* resistance has been found as shown in Tables 1 and 2. This previously unknown disease resistance arose from breeding and research efforts which were conducted beginning in 1988.

**TABLE 1**

| ***Phytophthora* Resistance in Periwinkle Cuttivars** | | |
|---|---|---|
| Cultivar | Resistant: Sensitive Ratio | |
| Pretty in Pink | 0:1 | (> 300)¹ |
| Pretty in White | 0:1 | (>300) |
| Pretty in Rose | 0:1 | (> 100) |
| Parasol | 0:1 | (> 100) |
| Tropicana Rose | 0:1 | (> 200) |
| Tropicana Bright Eye | 0:1 | (> 200) |
| Tropicana Pink | 0:1 | (> 200) |
| Tropicana Blush | 0:1 | (> 200) |
| Peppermint Cooler | 0:1 | (> 100) |
| Grape Cooler | 0:1 | (> 100) |
| Morning Mist | 0:1 | (> 100) |
| Pink Panther | 0:1 | ( 48) |
| Sahara Madness Bright Eye | 0:1 | ( 48) |
| Polka Dot | 0:1 | ( 48) |
| Little Pinkie | 0:1 | (> 300) |
| Little Bright Eye | 0:1 | (> 300) |
| Little Blanche | 0:1 | (> 300) |
| Little Delicata | 0:1 | ( 48) |
| Little Linda | 0:1 | ( 48) |
| Inter-variety hybrids² | 0:1 | (>2952) |

| | | |
|---|---|---|
| ¹Number In parentheses indicates number of individual plants tested. | | |
| ²123 hybrid combinations between commercially-available cultivars and Goldsmith breeding lines not containing *Phytophthora* resistance. Includes line numbers 13043, 13498, 13500, 13509, 13522, 13533, 13534, 13535, 13536, 13639, 13861, 14322, 14590 | | |

**TABLE 2**

| ***Phytophthora* Resistance in *Catharanthus* spp.** | | |
|---|---|---|
| Species | Source | Resistant : Sensitive Ratio |
| *C. longifolius* | Madagascar (8408)¹ | 0:1 |
| *C. roseus* | Madagascar (8430) | 0:1 |
| | Madagascar (8440) | 0:1 |
| | Madagascar (8442) | 0:1 |
| | Madagascar (8443) | 0:1 |
| | Mexico (13635)² | 0:1 |
| *C. trichophyllus* | Madagascar (12914)³ | 0:1 |

| | | |
|---|---|---|
| ¹Field collection number of R.N. Bowman | | |
| ²Provided by J. Marsh | | |
| ³Provided by A. Petit | | |

The instant invention is a genetic resistance to the disease *Phytophthora* in periwinkle wherein periwinkle plants which are exposed to this disease become infected but there is no spread or movement of this disease through the plant. The result is the resistant periwinkle plants stay healthy and do not die after exposure to *Phytophthora.* How this disease resistance gene acts by preventing the spread of the disease organism within a periwinkle plant is unknown. One possible mechanism of action is an enhanced alkaloid content within the plant.

The genetic basis for *Phytophthora* resistance in periwinkle seems to involve a single dominant allele. This observation parallels *Phytophthora* resistance observed in other crops as described in Erwin, et al. (1983). Ongoing breeding trials will help to further characterize the genetic basis for *Phytophthora* resistance in *Catharanthus.* When the disease resistance gene is incorporated into different genetic backgrounds of periwinkle, the disease resistance characteristic is transferred into these genetic backgrounds.

Once true-breeding resistant lines are developed that also possess horticulturally valuable traits (ex. compact habit, desirable colors), then open pollinated resistant seed from these lines can be marketed. Also possible is the use of a true-breeding resistant line as one of the parents in F₁ hybrid seed production resulting in an F₁ hybrid which expresses *Phytophthora* resistance. Additionally, transferring the *Phytophthora* resistance gene of the present invention into crops other than *Catharanthus* may reduce the damage caused by *Phytophthora* in those crops.

As used herein, the term "plant" includes plant cells, plant protoplasts, plant cells of tissue culture from which periwinkle plants can be regenerated, plant calli, plant clumps, and plant cells that are intact in plants or parts of plants such as pollen, flowers, seeds, leaves, stems, and the like.

Tissue culture of periwinkle is described in van der Heijden et al. "Cell and Tissue Cultures of *Catharanthus roseus* (L.) G. Don: A Literature Survey", Plant Cell, Tissue and Oman Culture 18:231-280 (1989), incorporated herein by reference. As described in this article, the presence of the therapeutically valuable cytotoxic alkaloids resulted in *Catharanthus* becoming one of the major fields of interest in modem plant cell biotechnology. The low yields of these dimeric indole alkaloids (approximately 0.0005%) and the subsequent high price of them were major motives to study the possibilities for the production of these alkaloids by cell and tissue cultures. The first efforts in this tissue culture with periwinkle date from approximately 25-30 years ago. Thus, another aspect of this invention is to provide for cells which upon growth and differentiation produce the disease resistance to *Phytophthora* in periwinkle.

In addition to providing resistance to *Phytophthora*, the transfer of the dominant resistance gene to different genetic backgrounds has produced what we believe are the associated characteristics of increased total alkaloid content expressed as a percent of total dry weight in the plant and increased tolerance or resistance to certain insects, including aphids and mites. It is believed that the elevated level of total alkaloid content may be directly associated with the increased plant resistance to aphids and mites and may also be somewhat associated with the level of *Phytophthora* resistance. The important traits of increased total alkaloid content and insect resistance are additional objects of the present invention.

Eilert et al., as discussed in Plant Physiol., 126:11 (1986); Arch. Biochem., Biophys., 254:491 (1987) were able to induce alkaloid accumulation in a cell suspension culture of *C. roseus* upon treatment of the cultures with homogenates of various fungi, thus further suggesting a relationship between alkaloid production and potential fungal resistance. Nef et al. Plant Cell Reports 10:26-29 (1991), were also able to stimulate major alkaloid production in *Catharanthus roseus* cells with an extract of the fungus *Pythium vexans.* Finally, vanDam, et al., Oecologia 95:425-430 (1993) found that indole alkaloid production in *Catharanthus roseus* was not inducible by mechanical leaf damage alone. The *Phytophthora* resistance gene of the instant invention is strongly implicated in the modulation of alkaloid synthesis and activity in *Catharanthus.* This activity, however, does not preclude other possible aspects of the resistance phenomenon.

Development of the *Phytophthora* resistance gene in *Catharanthus* has provided a straightforward means of providing non-pesticide protection to an important horticultural crop. Use of the gene imparts protection from ubiquitous *Phytophthora* infection and may aid in protection from other fungal and insect pathogens. Additionally, the gene is both user and environment friendly by reducing or eliminating the amounts of pesticides necessary to maintain the crop. Protection from *Phytophthora* as well as other potential broad spectrum protection makes the resistance gene potentially valuable in other crops. The annual dollar loss from *Phytophthora* infections is enormous. *Phytophthora cinnamomi,* alone, has nearly 1,000 hosts as discussed in Zentmeyer, Monogr. 10, Am. Phytopathol, Soc. 96 pp. (1980), many of which are important crop plants. *Phytophthora infestans*, the cause of the late blight of potatoes, which produced the great potato famine of 1846-47, resulted in the starvation of about 800,000 people. The disease is still a serious problem today. Non-pesticide reduction or elimination of *Phytophthora* losses would be of much significance to both food and ornamental crops. Any new source of resistance to either aerial or root *Phytophthora* diseases is potentially very valuable.

### EXAMPLES

The following examples are provided to further illustrate the present invention and are not intended to limit the invention beyond the limitations set forth in the appended claims.

### Example 1

### In Vitro Growth and Inoculation with the Phytophthora Pathogen

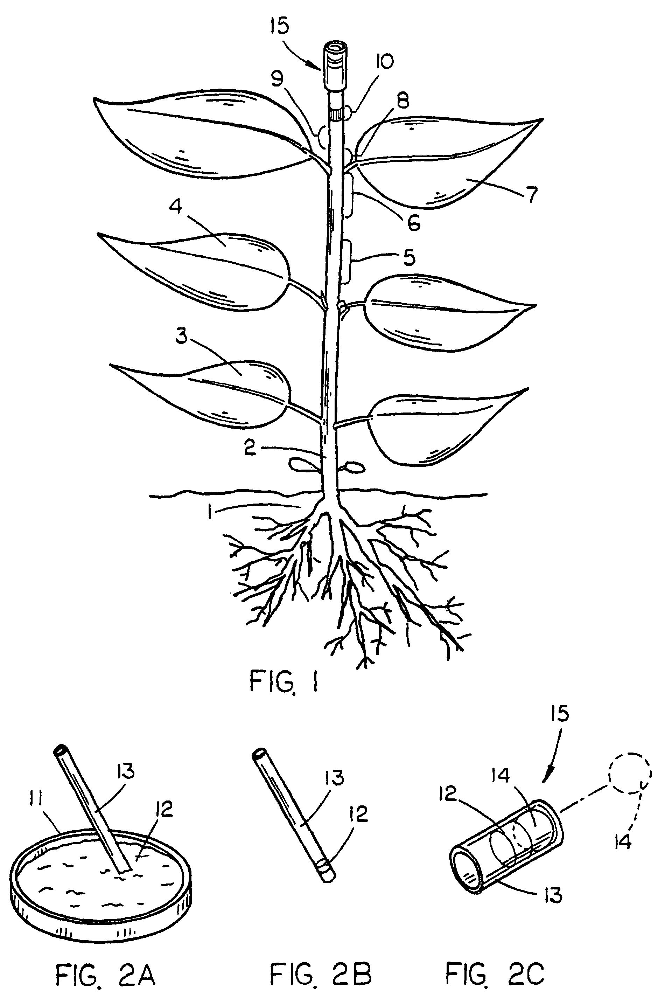

*In* vitro culture of *Phytophthora parasitica*, the causative agent of wilt in *Catharanthus*, is described in Gill, et al., (1977). The fungus readily grows on V-8 juice agar. Isolation and identification of strains of the fungus were accomplished by methods known to those skilled in the art. In testing for resistance to the fungus, we used the 12 different fungal isolates shown in Table 3 including pathogens obtained from commercial nurseries and plantings experiencing severe fungal attack. Isolates were obtained from Ferrin et al., as discussed in Ferrin et al., supra. Periwinkle plants were grown from seed using standard methods commonly known to those in the nursery trade. Plants were grown to the five-plus node stage (as shown in Figure 1) before inoculation. For comparisons of pathogenicity between the *Phytophthora* isolates, individual periwinkle plants were cloned via stem cuttings prior to inoculations.

Axenic *Phytophthora* cultures were used to prepare inoculation capsules as shown in Figures 2A, 2B, and 2C. A clear plastic drinking straw (13) was used to punch agar explants (12) of the fungus from the culture dish (11). The agar explant (12) was then positioned into a short inoculation capsule (15) where a 15 mm segment of the plastic drinking straw (13) was sealed at one end with a 6 mm. diameter spherical glass bead (14). The surface of the fungal mycelium was placed away from the bead.

Periwinkle seedlings were prepared by cutting the upright stem between the fourth and fifth nodes with a clean razor blade. As shown in Figure 1, an inoculation capsule (15) was then placed on the stem stump and gently pressed downward, thus forcing the fungal mycelium against the exposed stem surface. Controls were prepared in a similar manner, but without the fungal isolate.

The inoculated plants were then grown in a greenhouse using normal methods known to those skilled in the art. After approximately three weeks, the plants were then scored for the degree of movement of the fungus in the plant tissues.

**TABLE 3**

| **Sources of *Phytophthora parasitica* cultures**^{**1**} | |
|---|---|
| **Isolate** | **Source** |
| A | Premier Color Nursery, Fallbrook, CA |
| B | Premier Color Nursery, Fallbrook, CA |
| C | Premier Color Nursery, Fallbrook, CA |
| D | Premier Color Nursery, Fallbrook, CA |
| E | Premier Color Nursery, Fallbrook, CA |
| F | Premier Color Nursery, Fallbrook, CA |
| G | Premier Color Nursery, Fallbrook, CA |
| H | Summerlin Project, Summerlin Parkway, Las Vegas, NV |
| I | Summerlin Project, Summerlin Parkway, Las Vegas, NV |
| J | The Lakes Country Club, home garden, Palm Desert, CA |
| K | Color Spot Nursery, Fallbrook, CA |
| L | D. Ferrin, see Ferrin et al., 1992 |

| | |
|---|---|
| ¹Identity of all 12 isolates as *P. parasitica* was confirmed by Dr. J. Mitchell, Department Plant Pathology, University of Florida, Gainsville. | |

### Example 2

### Assessment of Infection - Definition of Resistance

The rating scale 1 - 10 as described in Table 4 and schematically shown in Figure 1 was used to measure the degree of infection and extent of movement of the pathogen through intact tissues of the inoculated plant. The degree of infection for each seedling in a family was determined as were the ratios of resistant to sensitive progeny as identified in Tables 1, 2, 5, 6 and 7. Three weeks after inoculation, the extent of tissue necrosis was assessed using the rating scale of Table 4 and Figure 1. Plants that were able to arrest the movement of the fungus within the uppermost portion of the top internode were considered resistant; additionally, plants showing partial necrosis at the uppermost node but without continued movement below the top-most node were considered resistant if the fungus did not continue downward nearing the second node. Resistance in *Catharanthus* to *Phytophthora parasitica* was defined as the ability to arrest the movement of the pathogen at or near the first node below the infection site. In contrast, sensitivity was defined as the inability to arrest the movement of the pathogen ultimately resulting in death of the plant.

**TABLE 4**

| **Rating Scale for Resistance Testing** | |
|---|---|
| Ratings of 5-10 are considered resistant. Ratings of 1-4 are considered sensitive or susceptible. | |

| Rating Scalez | Description of Category |
|---|---|
| | Also: See Figure 1 for Schematic Representation of Rating Scale |
| 10 | Clear Abscission zone formed well above uppermost node; no sign of darkening below abscission zone; abscission zone forming quickly. |
| 9 | Necrosis clearly stopped well above uppermost node; no abscission zone but no darkening (or continuation of darkening) well above uppermost node. |
| 8 | Necrosis stopped in crown at or just above uppermost node; no sign of infection in uppermost leaves. |
| 7 | One or both uppermost leaves chlorotic or clearly infected but not rapidly dying. The infection has been stopped at the node. |
| 6 | Infection present in uppermost portion of second intemode, appearing arrested just below the top node. |
| 5 | Compared to the sensitive control plants progression of necrosis is occurring much more slowly. Necrosis arrested within the second intemode, with no further movement, even after prolonged time. |
| 4 | Upper node dead, darkening of tower axils; several nodes clearly affected. No apparent arrest of the disease. |
| 3 | Two or more nodes dead; other nodes showing infection. |
| 2 | Some green in lower stem but death will occur in a few days. |
| 1 | Rapid and complete death, as in non-resistant controls. |

### Example 3

### Lack of Phytophthora Resistance in Existing Catharanthus Cultivars

All commercially available cultivars developed by public institutions and/or companies other than Goldsmith Seeds, Inc. were screened for *Phytophthora* resistance using the aforementioned methods. Results of the tests are shown in Table 1 and indicate that no commercial cultivar is resistant to *Phytophthora*. No commercially available cultivar is currently claimed by its manufacturer or distributor to be *Phytophthora* resistant. Chase and King, Grower Talks 57:63-69 (1993) have suggested that some cultivars are more susceptible to *Phytophthora* than others; they, however, found that all cultivars they tested were susceptible to *P.parasitica*.

### Example 4

### Lack of Phytophthora Resistance in Wild Catharanthus spp.

Assessions of *Catharanthus* spp. germplasm were also tested for *Phytophthora* resistance using the aforementioned methods of Examples 1 and 2. Wild germplasm of *Catharanthus* is difficult to obtain due to limited native distributions (Madagascar) and widespread destruction of its native habitat. Germplasm was collected in Madagascar of *C.longifolius and C. roseus.* Also representative germplasm of *C*.*trichophyllus* from Annik Petit (CNRS, Institut des Sciences Vegetates, France) was obtained. Non-resistance in these assessions is summarized in Table 2. Clearly, wild species of *Catharanthus* are not *Phytophthora* resistant.

### Example 5

### New Resistant Lines

As shown in Table 5, in the process of screening several hundred species x species, cultivar x species, and cultivar x cultivar *Catharanthus* hybrids for *Phytophthora* resistance in June, 1992, three experimental hybrid lines, 13516, 13517 and 13518 were identified that expressed good resistance. At that time, this resistance was the only resistance which we had ever found, despite concurrent extensive testing of all available native and naturalized collections and all existing cultivar germplasm. Further, the lines were not true breeding for resistance since they segregated both resistant and sensitive individuals as shown in Table 5. Subsequent analysis of pedigrees also shown in Table 5 suggested that the resistance may be the consequence of a single dominant (R) allele. Genetic analysis was complicated by the fact that parents in preceding generations had not been previously tested for resistance but had been discarded after the crosses were completed.

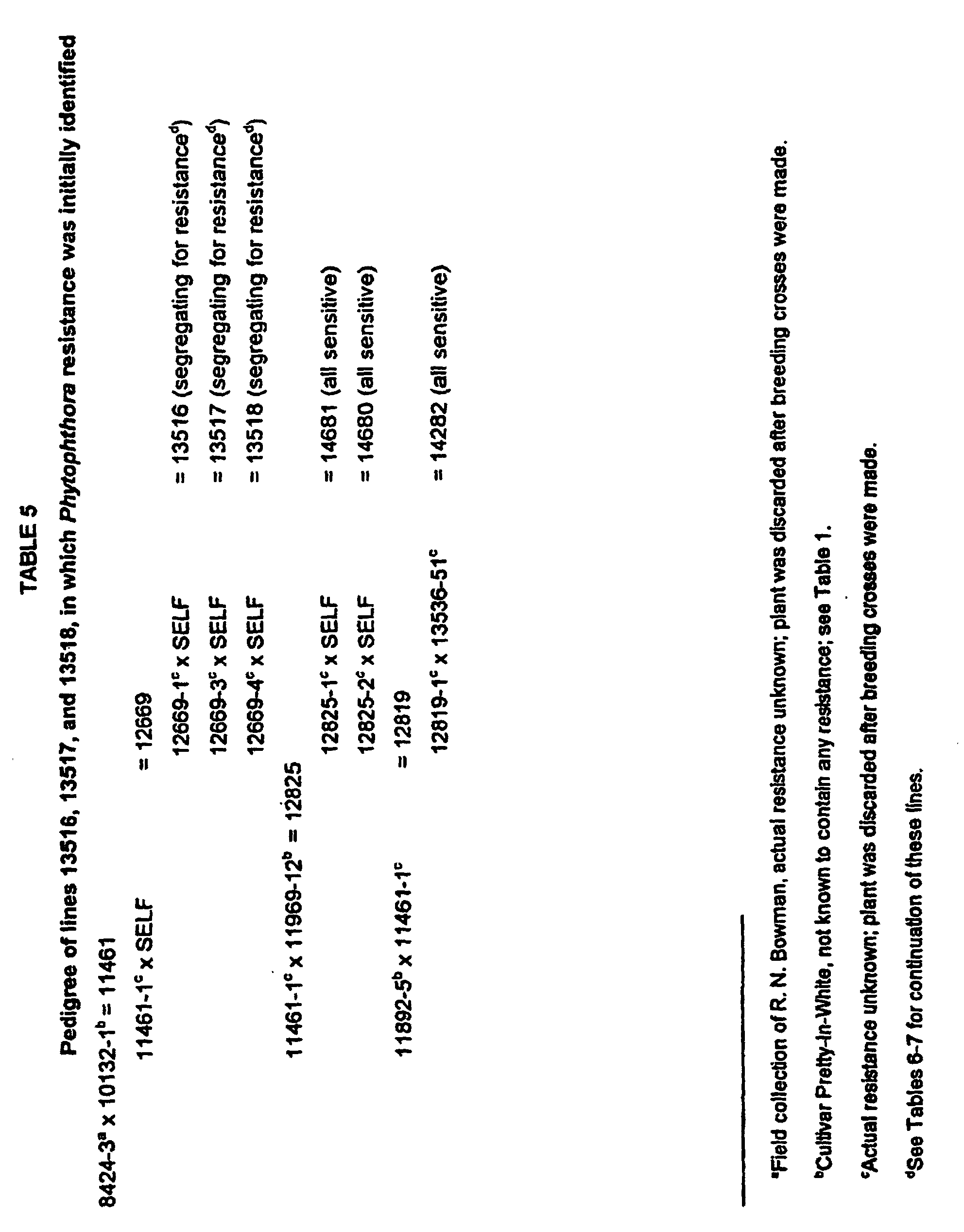

Pedigree analysis of the three lines carrying resistance (13516, 13517, 13518) indicated they shared in common a single plant (8424-3). This single plant had been considered a mutant form because of its light blue color and extreme infertility. We had never before seen the flower color in any wild species, cultivar or hybrid; further, the plant did not fit the key of any wild species as discussed in Veyret, *Catharanthus* Alkaloids, (1974). The initial motivation for using the plant in a breeding program was to capture the mutant color, remove it from its infertile background and use it for development of a new cultivar color.

Additional crossing generations were undertaken to better understand the genetic basis of the resistance gene.

### Example 6

### Genetic Segregation of Resistance

Individual plants from line numbers 13516, 13517, and 13518 were selfed; progenies in the next generation were checked for segregation of resistance (see Table 5). Additionally, as shown in Table 6, individuals from 13516, 13517, 13518 were also outcrossed to known sensitive lines. The F₁ progenies from these out crosses were also assessed for resistant: sensitive ratios.

The presence of sensitive progeny in selfs of phenotypically resistant individuals confirms that resistance is controlled by a dominant allele. Segregating ratios in selfs and F₁ generations confirm that resistance is monogenically controlled by two alleles, (R) for *Phytophthora* resistant, and (r) for *Phytophthora* sensitive. Table 7 indicates that F₂ segregations further substantiate the genetic basis for *Phytophthora* resistance in *Catharanthus.* In combination, Tables 5-7 further demonstrate the genetic basis for the resistance gene as a single dominant allele.

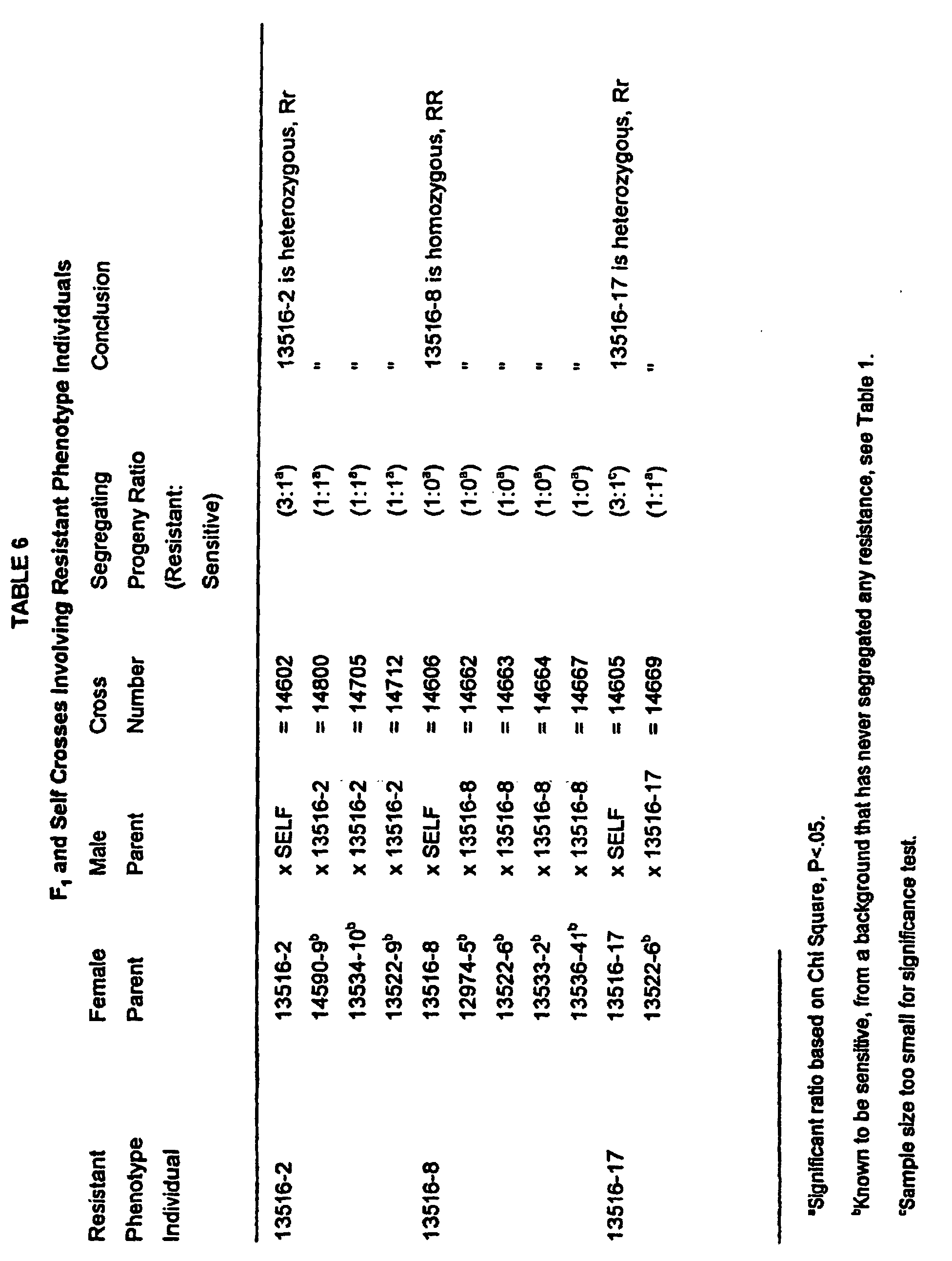

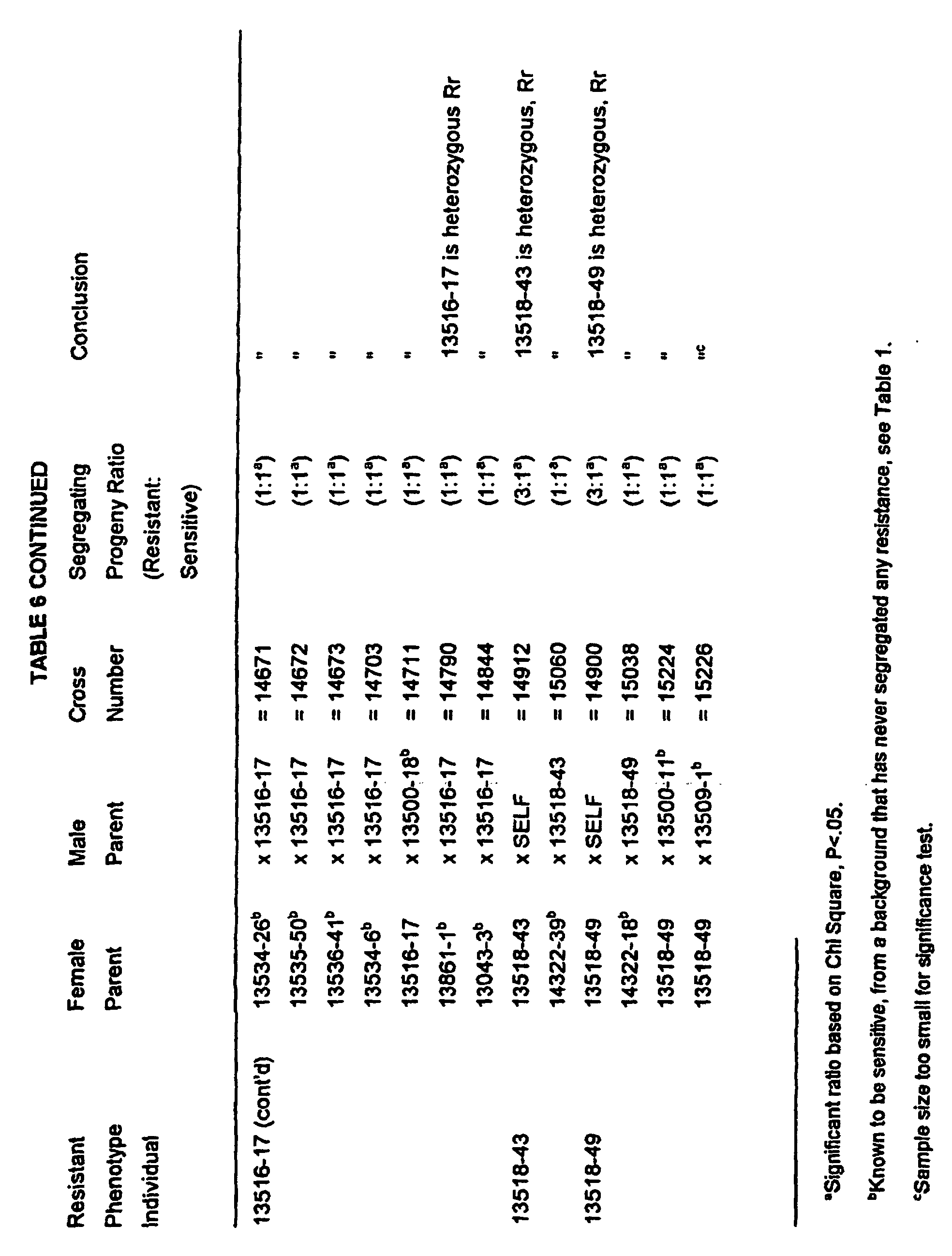

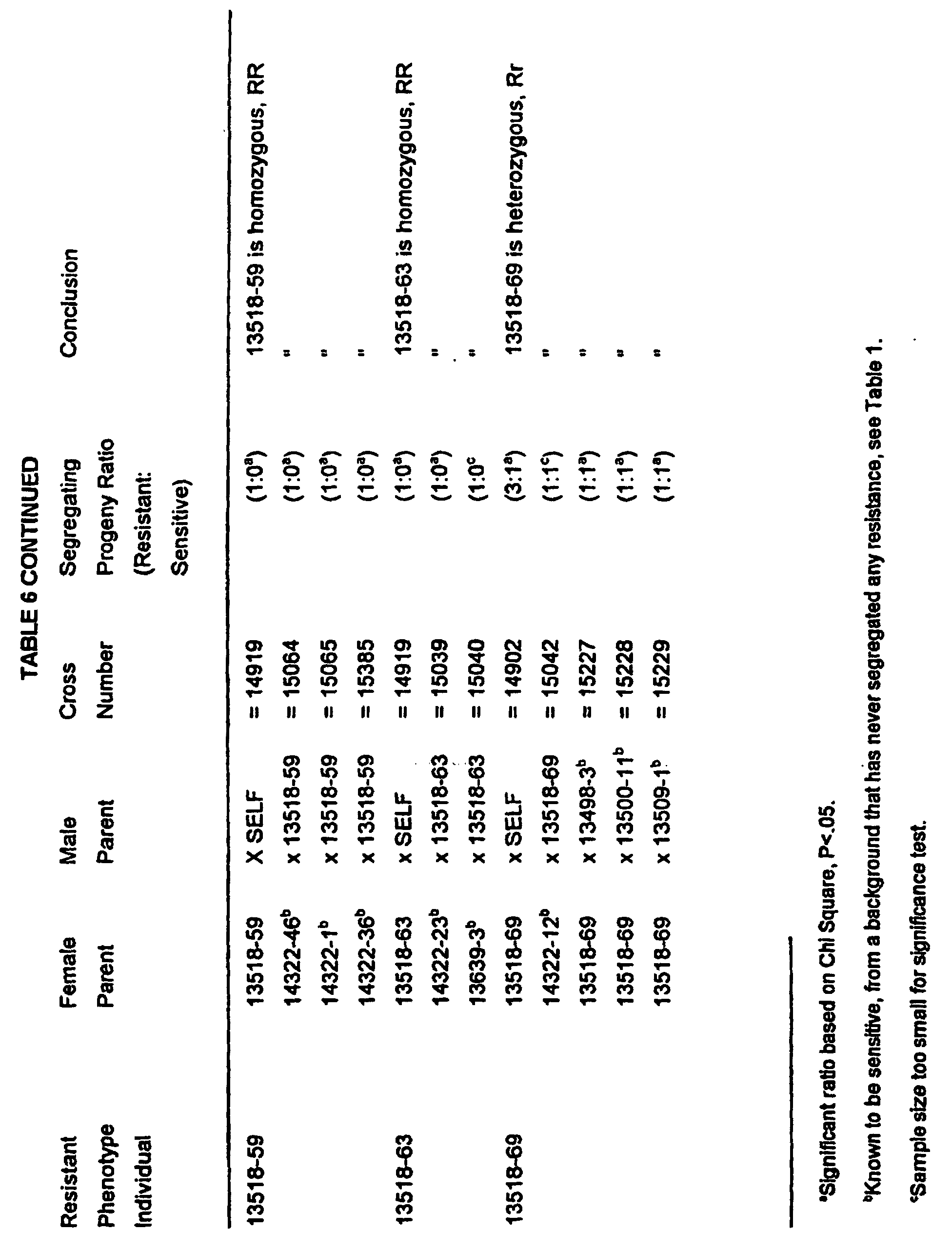

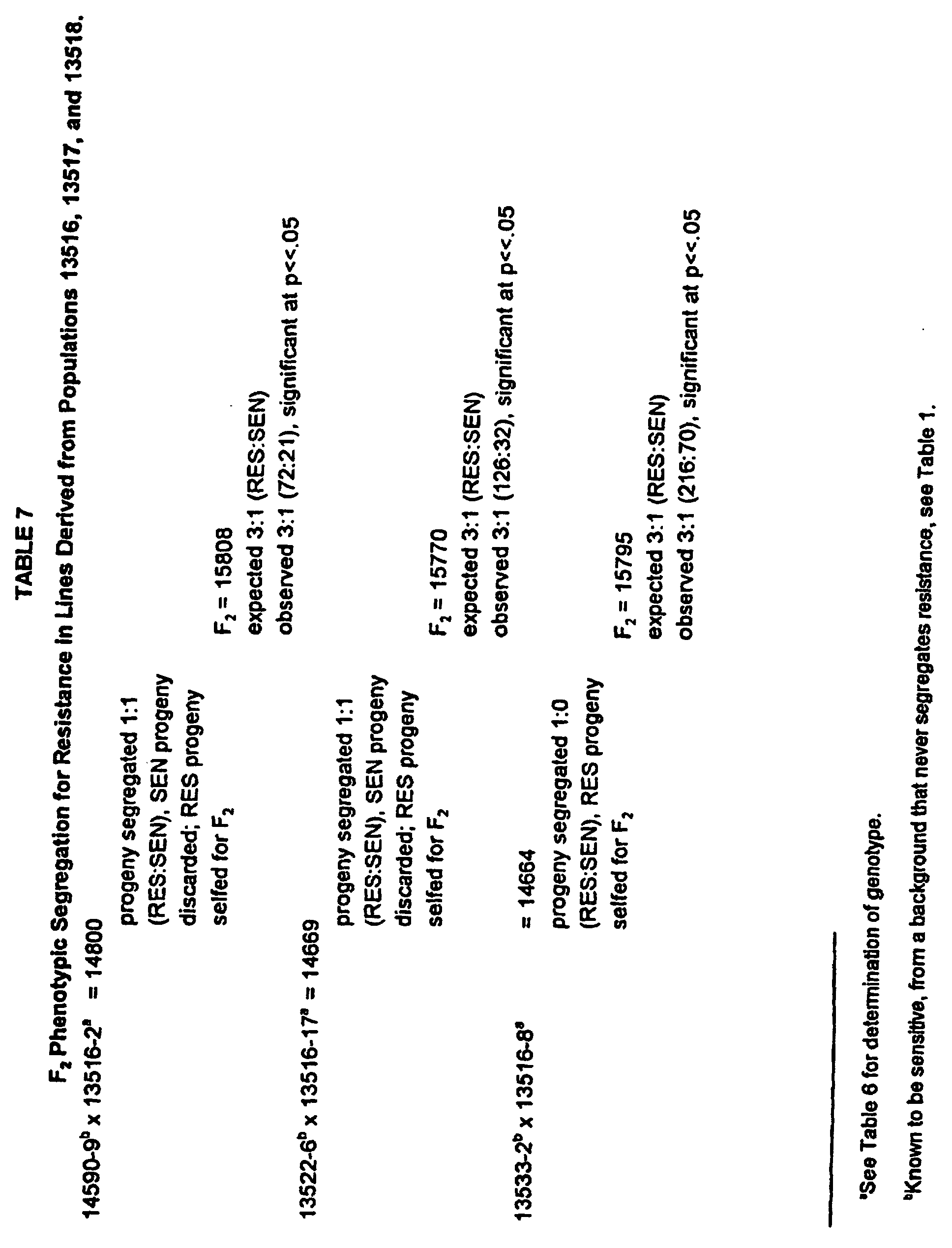

### Example 7

### Phytophthora Resistant Open-Pollinated Cultivars

As previously discussed, all existing commercial periwinkle cultivars are produced as open-pollinated crops. Flowers are primarily self pollinated in production fields or greenhouses and the resulting seed is harvested and sold. Open pollinated crops, including periwinkle, must be true breeding (homozygous) for desirable traits in order for expression of uniform continuity. Homozygous resistant (RR) lines have been developed which segregate only the resistant phenotype in all succeeding open-pollinated generations. The homozygous resistant lines now incorporate other homozygous horticulturally desirable traits including, but not limited to, plant habit, flower colors and flower size. Using conventional cross breeding methods, we have now crossed the *Phytophthora* resistance gene into all of the commercially significant periwinkle cultivars listed in Table 1 including the "Little", "Cooler", "Pretty In", and "Tropicana" series. Both the expression and segregation of the resistance gene in these diverse backgrounds were transferred as we had expected. This broad genetic base was used in the development of new *Phytophthora* resistant cultivars.

### Example 8

### Phytophthora Resistant F₁ Hybrid Cultivars

Even though all currently available commercial periwinkle cultivars are produced as open-pollinated crops, *Phytophthora* resistant F₁ hybrids also can be produced. In F₁ hybrids, a female line, incapable of pollinating itself either because it lacks pollen or is manually emasculated, is pollinated with pollen from another pollen-producing ("male") line. The resulting F₁ hybrid benefits from hybrid vigor and expresses aspects of both the pollen parent and female genomes. Other than periwinkle, a very large proportion of both the flower and vegetable seed, now sold is F₁ hybrid seed.

Periwinkle lines have been produced which are suitable for use as females in F₁ hybrid seed production. Because *Phytophthora* resistance is monogenically controlled by a dominant allele, either the male, female or both can provide *Phytophthora* resistance which will be expressed in the F₁ hybrid generations. Such **a** production scheme has resulted in new F₁ hybrid cultivars that are uniformly *Phytophthora* resistant.

### Example 9

### Additional Resistance to Other Pests including Aphids and Mites

Long term breeding programs for development of new periwinkle cultivars have provided ample opportunity to observe performance of existing cultivars and breeding lines with respect to sensitivity to common greenhouse pests including aphids *(Myzus persicae)* and mites *(Tetranychus urticae).* Table 8 indicates qualitative differences in susceptibility of selected lines and cultivars to these common greenhouse pests. Interestingly, lines selected for resistance to *Phytophthora* are less prone to attack by greenhouse pests such as aphids and mites. Conversely, lines known to be *Phytophthora* sensitive, are more prone to attack by aphids and mites. As shown in Table 8, line 13812 which was selected for extreme *Phytophthora* sensitivity is also extremely prone to aphid and mite infestations. Expression of the *Phytophthora* resistance gene of the instant invention appears to be strongly correlated with enhanced protection from other pests including, but not limited to, aphids and mites.

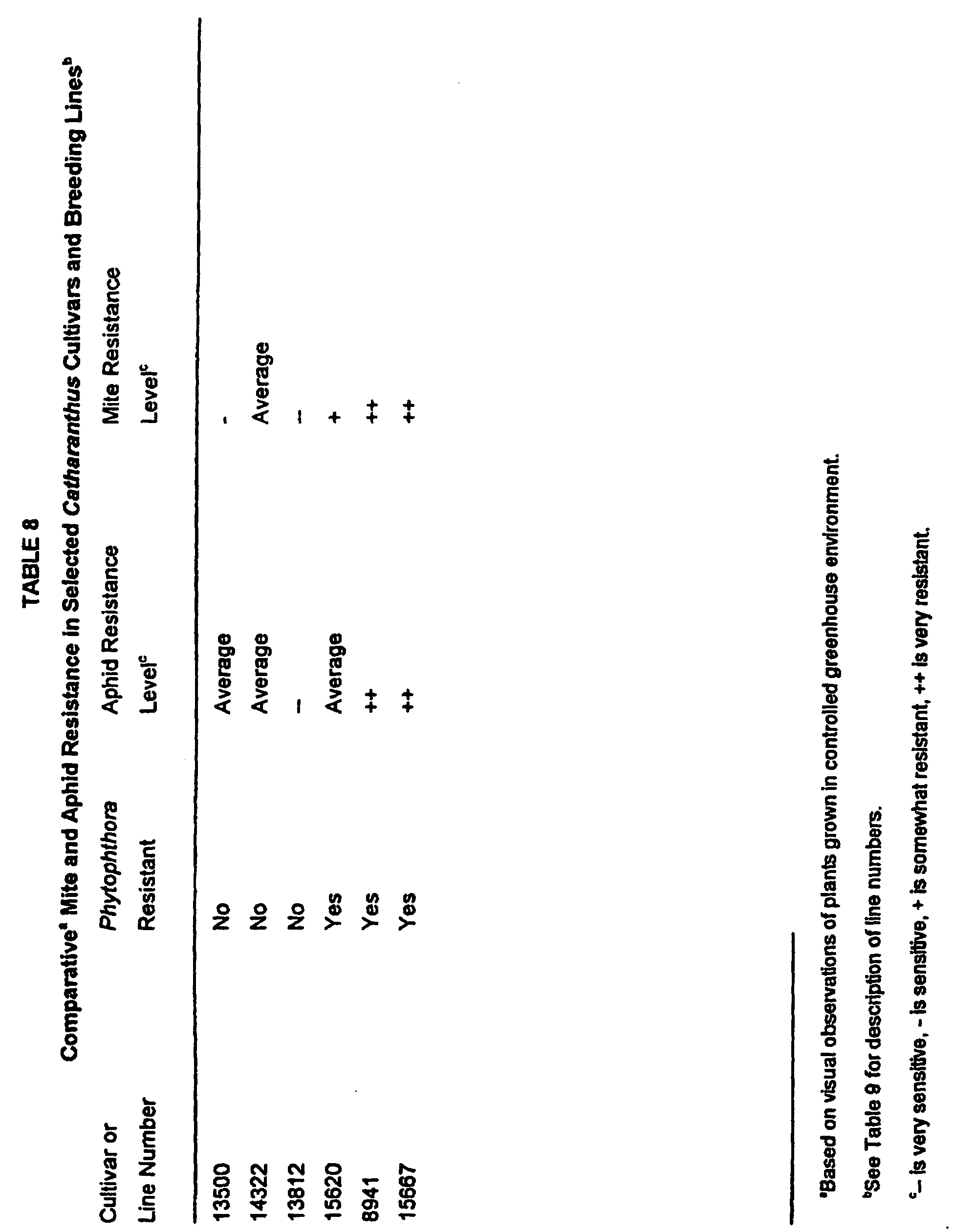

### Example 10

### Evaluation of Resistant Phenotypes - Possible Mechanism(s) of Action

As discussed prevously, *Catharanthus* species are well known for their production of alkaloids. Table 9 indicates the relationship between total alkaloid content and expression of *Phytophthora* resistance in selected *Catharanthus* lines. Line 15667, selected for *Phytophthora* resistance, contains more than 3.6 times the consensus level of total alkaloids reported for any other *Catharanthus* species or cultivar. Line 15667 is also highly resistant to aphids and mites as shown in Table 8. Indirect evidence thus indicates that the mode of action of the *Phytophthora* resistance gene involves enhanced alkaloid production and/or activity. Other lines such as 15614 and 15620 in Table 9, do not have elevated total alkaloid levels, yet still express *Phytophthora* resistance, thus suggesting that qualitative changes in alkaloid composition, rather than total alkaloid content, may have effected the resistance phenotype. The *Phytophthora* resistance gene of the instant invention is strongly implicated in the modulation of alkaloid synthesis and activity in *Catharanthus.* This activity, however, does not preclude other possible aspects of the resistance phenomenon.

### Example 11

### Transformation of Other Crop Species with the Resistance Gene to Provide Pest Resistance

The direct applicability of using the *Phytophthora* resistance gene of the present invention in transformation of other crops species sensitive to *Phytophthora* diseases is significant. Transformation methods now applied to many plant species (cf. Robinson and Riroozabady, Scientia Horticulturae 55:83-99, 1993) enable the movement of desirable genes from a source (in this case, *Phytophthora* resistant *Catharanthus*) to a target species. Using currently available molecular methods known to those in the art, the gene is sequenced, cloned and inserted in other species where its expression is valuable. Expression of the resistance gene in crops now sensitive to aerial *Phytophthora* diseases including palms, *Bougainvillea* and other ornamentals provide a new source of non-pesticide protection. At first glance, one might suspect that heightened alkaloid levels (and thus *Phytophthora* resistance) might interfere with food crops. While this situation is possible, potatoes provide a good example of how food crops will benefit from transformational insertion of our resistance gene; potato stems and leaves are already toxic due to high levels of alkaloids they contain, yet tubers produced by the plants are edible. Thus, altered alkaloid expression, accomplished through transformation with the resistance gene of the present invention may impart additional non-pesticide crop protection without affecting the food quality of the crop.

Selected genes have now been isolated and cloned from *Catharanthus roseus.* Meijer, et al., Plant Mol. Biol., 22:379-383 (1993), incorporated herein by reference, isolated cytochrome P-450 cDNA clones from *C. roseus* using standard methods known to those skilled in the art. Goddijin, et al., Plant Mol. Biol. 22:907-912 (1993) incorporated herein by reference, isolated a tryptophan decarboxylase gene from *C. roseus*, and used this same gene as a selectable marker in *Agrobacterium*-mediated tobacco leaf disc transformation experiments. They were able to recover mature transformed tobacco plants expressing the *Catharanthus* gene. Methods are, thus, currently available to those skilled in the art, for transformations using our *Phytophthora* resistance gene in other crop species.

### Example 12

### Commercial Alkaloid Production Use of the Resistance Gene to Modulate Alkaloid Quantity and Quality

Selection for resistance to *Phytophthora* has resulted in an increased level of alkaloids in selected lines. Importantly, however, it should be noted that these lines were not specifically selected for alkaloid content or quality. Since it is now apparent that the resistance gene directly influences the total amount of alkaloids produced, as shown in Table 9, one can intentionally select for increased alkaloid content by screening lines that contain the resistance gene. Yoder and Mahlberg Am. J. Bot., 63:1167-1173 (1976) suggested that the high concentration ("1.5%") of indole alkaloids in leaves of *Catharanthus roseus* and the fact that they are mainly found in specialized cells indicate that the plant stores indole alkaloids constitutively and probably has already evolved mechanisms to overcome auto-intoxication. It is expected that elevated alkaloid contents can be attained which exceed those reported in Table 9; further, selection for specific alkaloids rather than total alkaloid content results in an effective means of improving alkaloid production in *Catharanthus*. Currently, we are not aware of any U.S. patents covering alkaloid synthesis genes in *Catharanthus*; additionally, the synthetic pathways leading to the significant alkaloids including catharanthine, vindoline, vinblastine and vincristine are only reported from *Catharanthus.* Quantitatively, catharine, vindoline and anhydrovinblastine are the alkaloids in greatest abundance in *Catharanthus* as discussed in Balsevich and Hogge, J. Nat. Prod. 51:1173-1177. Field grown plants of *Catharanthus roseus* are the only commercial source (at present) for the production of anti-cancer therapeutic alkaloids vinblastine and vincristine. Given the dollar value of periwinkle alkaloids, even small improvements in alkaloid quantity or quality can translate to enormous economic potential. Over a long period of time, numerous attempts have been made to use tissue culture methods for *in vitro* production of alkaloids. Economic efficiency of these *in vitro* methods has not been achieved, in part due to the low amounts of alkaloids produced. Use of the *Phytophthora* resistance gene to increase levels of alkaloid production and/or quality can make *in vitro* alkaloid production feasible. For this reason, the resistance gene can be used in whole plants, plant organs, tissues, cells, protoplasts or cell extracts for any aspect of *Catharanthus* alkaloid production. Plants containing the genetic resistance allele of the present invention are grown under field production conditions to obtain the desired alkaloid production.

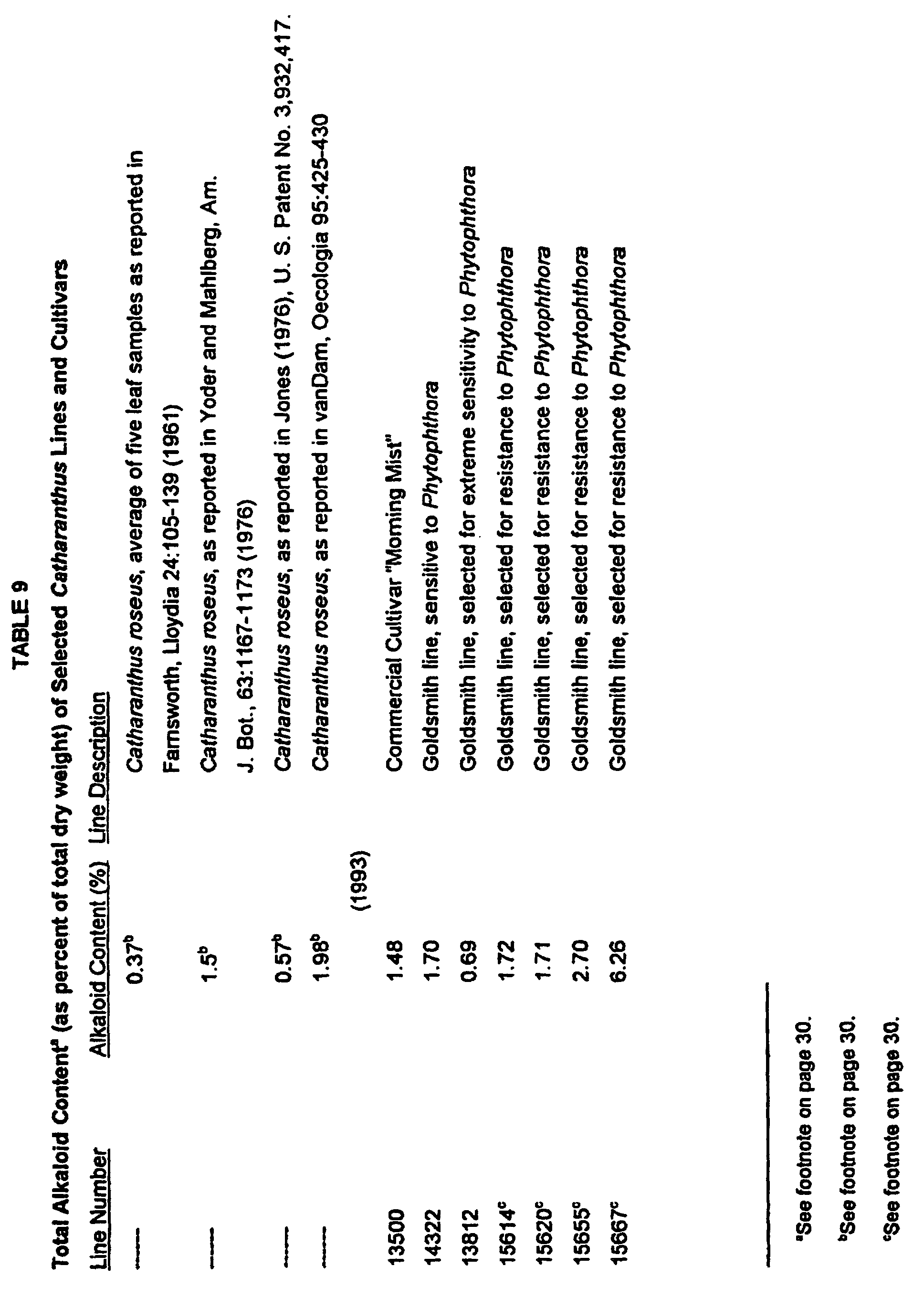

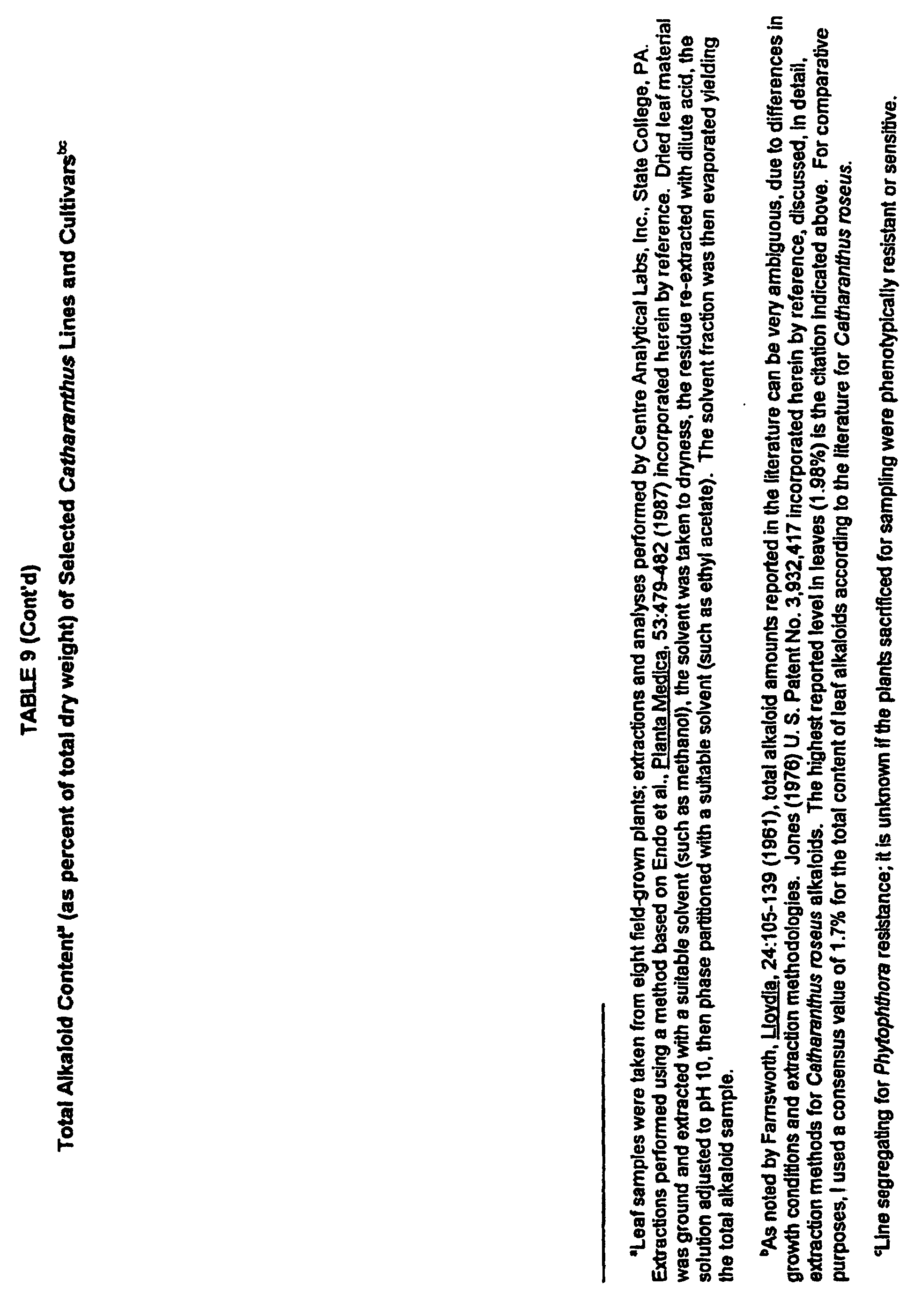

### DEPOSIT INFORMATION

Periwinkle seeds have been placed on deposit with the American Type Culture Collection (ATCC), Rockville, Md. 20852, under Deposit Accession Number 75636 on January 14, 1994.

Although the foregoing invention has been described in some detail by way of illustration and example for purposes of clarity and understanding, it will be obvious that certain changes and modifications may be practiced within the scope of the invention, as limited only by the scope of the appended claims.

## Claims

1. A *Catharanthus* seed containing a genetic resistance to *Phytophthora.*

2. The *Catharanthus* seed of claim 1, wherein said seed contains a dominant allele for resistance to *Phytophthora parasitica.*

3. A *Catharanthus* plant produced by growing the seed of claim 1.

4. Pollen of the plant of claim 3.

5. An ovule of the plant of claim 3.

6. A tissue culture comprising regenerable cells of the plant of claim 3, wherein said plant contains a genetic resistance to *Phytophthora.*

7. A method for producing F₁ hybrid *Catharanthus* seed comprising crossing a first parent *Catharanthus* plant with a second parent *Catharanthus* plant and harvesting the resultant F₁ hybrid *Catharanthus* seed, wherein said first or second parent *Catharanthus* plant is the *Catharanthus* plant of claim 3.

8. A *Catharanthus* plant according to claim 3, having a total alkaloid content of about 2.0% or greater.

9. A *Catharanthus* plant according to claim 8, wherein said total alkaloid content is between about 2.0% and about 6.5%.

10. A method for producing a *Catharanthus* seed having a total alkaloid content of 2.0% or greater comprising the steps of:
a) planting in pollinating proximity seeds of a *Catharanthus* genotype containing a genetic resistance to *Phytophthora* and another *Catharanthus* genotype;
b) cultivating *Catharanthus* plants resulting from said seeds until said plants bear flowers;
c) emasculating the male flowers of the plants of either *Catharanthus* genotype;
d) allowing cross pollination to occur between said *Catharanthus* genotypes; and,
e) harvesting seeds produced on said emasculated plants of the *Catharanthus* line.

11. A first generation (F₁) hybrid *Catharanthus* plant produced by growing said hybrid *Catharanthus* seed of claim 7 or 10.

12. A viable *Catharanthus* seed according to claim 1 or a viable *Catharanthus* plant according to claim 3 and succeeding generations thereof grown from the seeds deposited under ATCC Accession No. 75636 and *Catharanthus* seeds and plants to which the *Phytophthora* resistance allele is transferred from said deposited seeds or succeeding generations thereof.

13. A method for transferring a genetic *Phytophthora* resistance as defined in claim 1 from *Catharanthus* to another species comprising the steps of:
a) isolating said genetic *Phytophthora* resistance gene;
b) combining said genetic *Phytophthora* resistance gene with a promoter; and,
c) inserting the genetic *Phytophthora* resistance gene into another species using a transformation technique.

14. A *Catharanthus* plant regenerated from said tissue culture of claim 6.

## Patentansprüche

1. *Catharanthus*-Samen, enthaltend eine genetische Resistenz gegen *Phytophthora*.

2. *Catharanthus*-Samen von Anspruch 1, wobei der Samen ein dominantes Allel für Resistenz gegen *Phytophthora parasitica* enthält.

3. *Catharanthus*-Pflanze, hergestellt durch Aufzucht des Samens von Anspruch 1.

4. Pollen der Pflanze von Anspruch 3.

5. Eizelle der Pflanze von Anspruch 3.

6. Gewebekultur, umfassend regenerierbare Zellen der Pflanze von Anspruch 3, wobei die Pflanze eine genetische Resistenz gegen *Phytophthora* enthält.

7. Verfahren zur Herstellung eines F₁-Hybrid*-Catharanthus-*Samens, umfassend das Kreuzen einer ersten Eltern-*Catharanthus*-Pflanze mit einer zweiten Eltern-*Catharanthus*-Pflanze und Ernten des resultierenden F₁-Hybrid-*Catharanthus*-Samens, wobei die erste oder zweite Eltern-*Catharanthus-*Pflanze die *Catharanthus*-Pflanze von Anspruch 3 ist.

8. *Catharanthus-*Pflanze gemäß Anspruch 3, welche einen Gesamt-Alkaloid-Gehalt von etwa 2,0 % oder mehr aufweist.

9. *Catharanthus-*Pflanze gemäß Anspruch 8, wobei der Gesamt-Alkaloid-Gehalt zwischen etwa 2,0 % und etwa 6,5 % liegt.

10. Verfahren zur Herstellung eines *Catharanthus*-Samens mit einem Gesamt-Alkaloid-Gehalt von 2,0 % oder mehr, umfassend die folgenden Schritte:
a) In Pollenflugweite erfolgendes Einpflanzen von Samen eines *Catharanthus-*Genotyps, enthaltend eine genetische Resistenz gegen *Phytophthora,* und eines weiteren *Catharanthus*-Genotyps;
b) Kultivieren von aus den Samen resultierenden *Catharanthus*-Pflanzen, bis die Pflanzen Blüten tragen;
c) Entmannen der männlichen Blüten der Pflanzen jedweden *Catharanthus*-Genotyps;
d) Ermöglichen des Auftretens von Kreuz-Bestäubung zwischen den *Catharanthus*-Genotypen; und
e) Ernten der auf den entmannten Pflanzen der *Catharanthus-*Linie erzeugten Samen.

11. (F₁)Hybrid*-Catharanthus*-Pflanze der ersten Generation, hergestellt durch Aufziehen des Hybrid*-Catharanthus*-Samens von Anspruch 7 oder 10.

12. Lebensfähiger *Catharanthus*-Samen gemäß Anspruch 1 oder lebensfähige *Catharanthus*-Pflanze gemäß Anspruch 3 und nachfolgende Generationen davon, herangezüchtet aus den Samen, hinterlegt unter der ATCC-Zugangsnummer 75636, und *Catharanthus-*Samen und -Pflanzen, auf welche das *Phytophthora-*Resistenzallel aus den hinterlegten Samen übertragen wurde, oder nachfolgende Generationen davon.

13. Verfahren zur Übertragung einer genetischen *Phytophthora*-Resistenz, wie definiert in Anspruch 1, von *Catharanthus* auf eine andere Spezies, umfassend die folgenden Schritte:
a) Isolieren des genetischen *Phytophthora*-Resistenzgens:
b) Kombinieren des genetischen *Phytophthora-*Resistenzgens mit einem Promotor; und
c) Einführen des genetischen *Phytophthora*-Resistenzgens in eine andere Spezies unter Anwendung einer Transformationstechnik.

14. *Catharanthus*-Pflanze, regeneriert aus der Gewebekultur von Anspruch 6.

## Revendications

1. Graine de *Catharanthus* contenant une résistance génétique à *Phytophtora.*

2. Graine de *Catharanthus* de la revendication 1, ladite graine contenant un allèle dominant pour la résistance à *Phytophtora parasitica.*

3. Plante de *Catharanthus* produite en faisant pousser la graine de la revendication 1.

4. Pollen de la plante de la revendication 3.

5. Ovule de la plante de la revendication 3.

6. Culture de tissu comprenant des cellules pouvant être régénérées de la plante de la revendication 3, où ladite plante contient une résistance génétique à *Phytophtora.*

7. Méthode de production d'une graine de *Catharanthus* hybride F₁ comprenant le fait de croiser une première plante parent de *Catharanthus* avec une seconde plante parent de *Catharanthus* et de récolter la graine de *Catharanthus* hybride F₁ résultante, dans laquelle ladite première ou seconde plante parent de *Catharanthus* est la plante de *Catharanthus* de la revendication 3.

8. Plante de *Catharanthus* selon la revendication 3, ayant une teneur totale en alcaloïdes d'environ 2,0 % ou plus.

9. Plante de *Catharanthus* selon la revendication 8, dans laquelle ladite teneur totale en alcaloïdes est entre environ 2,0 % et environ 6,5 %.

10. Méthode de production d'une graine de *Catharanthus* ayant une teneur totale en alcaloïdes de 2,0 % ou plus comprenant les étapes consistant :
a) à planter à une proximité permettant la pollinisation des graines d'un génotype de *Catharanthus* contenant une résistance génétique à *Phytophtora* et d'un autre génotype de *Catharanthus* ;
b) à cultiver des plantes de *Catharanthus* réultant desdites graines jusqu'à ce que lesdites plantes portent des fleurs ;
c) à émasculer les fleurs mâles des plantes de l'un des génotypes de *Catharanthus ;*
d) à laisser se produire une pollinisation croisée entre lesdits génotypes de *Catharanthus* ; et
e) à récolter les graines produites sur lesdites plantes émasculées de la lignée de *Catharanthus.*

11. Plante de *Catharanthus* hybride de première génération (F₁) produite en faisant pousser ladite graine de *Catharanthus* hybride de la revendication 7 ou 10.

12. Graine de *Catharanthus* viable selon la revendication 1 ou plante de *Catharanthus* viable selon la revendication 3 et générations suivantes de celles-ci que l'on a fait pousser à partir des graines déposées sous le numéro d'accès ATCC No. 75636 et de graines et de plantes de *Catharanthus* auxquelles on a transféré l'allèle de résistance à *Phytophtora* à partir desdites graines déposées ou de leurs générations suivantes.

13. Méthode pour transférer une résistance génétique à *Phytophtora* comme défini dans la revendication 1 de *Catharanthus* à une autre espèce comprenant les étapes consistant :
a) à isoler ledit gène de résistance génétique à *Phytophtora ;*
b) à combiner ledit gène de résistance génétique à *Phytophtora* avec un promoteur ; et
c) à insérer le gène de résistance génétique à *Phytophtora* dans une autre espèce en utilisant une technique de transformation.

14. Plante de *Catharanthus* régénérée à partir de ladite culture de tissu de la revendication 6.
